(19) Europäisches Patentamt
European Patent Office
Office européen des brevets

(11)  **EP 4 349 301 A1**

(12)  **EUROPEAN PATENT APPLICATION**
published in accordance with Art. 153(4) EPC

(43) Date of publication:
**10.04.2024   Bulletin 2024/15**

(21) Application number: **21943174.9**

(22) Date of filing: **28.05.2021**

(51) International Patent Classification (IPC):
**A61C 13/00** (2006.01)   **A61C 13/083** (2006.01)
**A61K 6/802** (2020.01)   **A61K 6/853** (2020.01)
**A61L 27/10** (2006.01)   **C04B 35/622** (2006.01)
**C04B 35/14** (2006.01)   **C04B 35/01** (2006.01)

(52) Cooperative Patent Classification (CPC):
**A61C 13/00; A61C 13/083; A61K 6/802;
A61K 6/853; A61L 27/10; C04B 35/01;
C04B 35/14; C04B 35/622**

(86) International application number:
**PCT/KR2021/006675**

(87) International publication number:
**WO 2022/250185 (01.12.2022 Gazette 2022/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: Hass Co., Ltd.
**Gangwon-do 25452 (KR)**

(72) Inventors:
• **LIM, Hyung Bong
  Ansan-si, Gyeonggi-do 15521 (KR)**
• **KIM, Yong Su
  Gangneung-si, Gangwon-do 25497 (KR)**

(74) Representative: **Gulde & Partner
Patent- und Rechtsanwaltskanzlei mbB
Wallstraße 58/59
10179 Berlin (DE)**

(54)  **DENTAL BULK BLOCK FOR MACHINING AND METHOD FOR MANUFACTURING SAME**

(57)  Disclosed is a dental bulk block for machining, which is a glass ceramic block including a crystalline phase in an amorphous glass matrix, the dental bulk block being a functionally graded material including a crystalline phase in which the main crystalline phase is lithium disilicate and the additional crystalline phase comprises at least one selected from cristobalite and tridymite, and quartz and lithium phosphate, and having a main crystalline size gradient with respect to depth and no interface present at the point of change in the main crystalline size gradient. Thus, the dental bulk block is useful for the manufacture of artificial tooth prostheses that are similar to natural teeth, and accordingly, can not only reduce time and processes for manufacturing an artificial tooth prosthesis, but can also provide a structural stability enhancement effect in terms of force distribution due to functionally graded mechanical characteristics.

**EP 4 349 301 A1**

**Description**

**Technical Field**

The present disclosure relates to a dental bulk block for machining, which is useful in manufacturing artificial teeth having structural properties similar to those of natural teeth, and a method of manufacturing the same. The present disclosure is the result of research conducted with support from Gangwon TechnoPark on the Material-Parts-Equipment Business Development and Technology Independence Support Project.

**Background Art**

Crown materials refer to prosthetic materials that restore the dentin and enamel of damaged teeth and can be classified into inlays, onlays, veneers, crowns, and the like depending on application sites. Crown materials restore the outer surface of teeth and thus typically require aesthetic properties. In addition, due to fractures such as chipping or wearing between the opposing teeth, high strength is required. Examples of existing crown materials used include leucite glass-ceramics, reinforced porcelain, and fluorapatite ($Ca_5(PO_4)_3F$) glass-ceramics, which have excellent aesthetic properties but are disadvantageous in that fractures are likely to occur due to the low strength of 80 to 120 MPa. Accordingly, research is currently in progress on developing high-strength crown materials made of various materials.

Lithium silicate glass-ceramic was introduced in 1973 by Marcus P. Borom and Anna M. Turkalo (The Pacific Coast Regional Meeting, The American Ceramic Society, San Francisco, CA, October 31, 1973 (Glass division, No.3-G-73P)).

Crystalline phases and strength were studied for a variety of crystal nucleation and growth under heat-treatment conditions using $Li_2O$-$Al_2O_3$-$SiO_2$-$Li_2O$-$K_2O$-$B_2O_3$-$P_2O_5$-based glass. The crystalline phase of high-temperature lithium disilicate formed from low-temperature lithium metasilicate exhibits a strength of 30 to 35 KPS. This was due to the residual stress attributed to the difference in thermal expansion coefficients between base glass, mother glass, $Li_2SiO_5$, and $Li_2SiO_3$ phases.

Materials and methods for manufacturing artificial teeth (monolithic dental crowns) using glass containing lithium disilicate crystals have already been known in several patents. However, in such known technologies, crystalline phases have a large size, and instant machining is thus difficult. For machining, a method of forming a high-strength lithium disilicate crystalline phase by primarily forming and machining a lithium metasilicate crystalline phase (machinable crystalline) and then secondarily performing heat treatment is used. However, in this case, the shrinkage caused by the post-heat treatment process makes dimensional accuracy poor, and there is the hassle of adding the heat treatment process. Typically, the machining of prostheses using CAD/CAM requires a hospital to directly machine a bulk material to manufacture a prosthesis so that the manufactured prosthesis is fitted for the patient as quickly as possible (one-day appointment). Thus, the delay in time due to the heat treatment process adds a financial burden to patients and users.

In addition, the existing lithium disilicate glass-ceramic materials have limitations in realizing high optical transmittance or opalescence similar to that of natural teeth due to the large crystalline phase.

In particular, existing lithium disilicate glass-ceramic materials are prepared by primarily forming well-machinable lithium metasilicate glass-ceramics for machining and then forming lithium disilicate through secondary crystallization heat treatment after the machining to improve strength. In this case, the crystalline phase had a size of about 3 $\mu$m or more, so the machinability was significantly poor in this state, and only the strength was realizable.

To solve this problem, the applicants of the present disclosure proposed a method of manufacturing a glass-ceramic containing a well-machinable lithium disilicate crystalline phase and a silicate crystalline phase by controlling a crystal size with varying primary heat-treatment temperatures and received a patent for the method (Korean Patent No. 10- 1975548). Specifically, in this case, disclosed was a method of manufacturing a dental glass-ceramic containing a silica crystalline phase. The method includes: performing primary heat treatment on a glass composition at a temperature of 400°C to 850°C, the glass composition containing 60 to 83 wt% of $SiO_2$, 10 to 15 wt% of $Li_2O$, and 2 to 6 wt% of $P_2O_5$ serving as a nucleation agent, 1 to 5 wt% of $Al_2O_3$ to increase a glass transition point and softening point and to enhance glass chemical durability, 0.1 to 3 wt% of SrO to increase a glass softening point, 0.1 to 2 wt% of ZnO, 1 to 5 wt% of a colorant, and 2.5 to 6 wt% of $Na_2O$ plus $K_2O$, which are alkali metal oxides, to increase a glass thermal expansion coefficient; and performing secondary heat treatment at a temperature of 780°C to 880°C after the primary heat treatment. The method is characterized in that a silica crystalline phase and a nano-sized lithium disilicate crystalline phase having a size of 5 nm to 2000 nm are generated through the primary heat treatment, and the transmittance is controlled by the secondary heat treatment temperature.

On the other hand, as the living standards of humans are improved, there is a growing demand for aesthetics in the field of dentistry. With the growing needs of patients for aesthetics, much research has been conducted on aesthetic prosthetic restorations using various materials.

Factors that affect the aesthetic properties of a ceramic restoration, which is a currently and mainly available aesthetic restorative material, include the appearance of a tooth, surface condition, transparency, shade, and the like. Among these factors, transparency is regarded as one of the key factors for the successful manufacturing of restorations. Although the mechanical and physical properties of such ceramic materials for aesthetic prostheses have been studied and developed, there are still many problems with the combination of shades. In addition, from a clinical and technical view, the selection of shade for restorations, especially transparency, has many difficulties.

In aesthetic prosthodontics, factors that affect aesthetic properties during tooth restoration include shades (colors), tooth shapes and sizes, tooth arrangements and proportions, light rays, transmittance, and the design of restorations. In reality, tooth colors and shapes are the factors to which our eyes are sensitive.

There is no part in natural teeth where color is the same from the cervical region to the incisal edge of the teeth.

Based on this point, a method of manufacturing an artificial tooth capable of mimicking deep colors of natural teeth using a so-called build-up method has also been recently known.

The build-up method is a method of staking layers of powder, such as porcelain or zirconia, to form a tinted artificial tooth and performing heat treatment to realize layers of shades similar to those of natural teeth. Although the color of natural teeth is mimicable to a significant level using the build-up method, the aesthetics of the artificial tooth are completely determined by the skill of technicians. Thus, there are problems of poor reproducibility, being not beneficial to patients because the instant manufacturing thereof is impossible, and being difficult to be implemented by a machining method, such as CAD/CAM.

On the other hand, when manufacturing artificial teeth by a machining method, such as CAD/CAM, using existing bulk blocks, the bulk blocks themselves are made of materials exhibiting uniform physical properties. Thus, the resulting artificial teeth, unlike natural teeth, have no choice but to be obtained in a form tinted with a single shade. In particular, when applying artificial teeth, according to this method, to front teeth and the like, there is bound to be a problem in that aesthetic heterogeneity makes the teeth look less natural.

The transparency and machinability can be controlled through the secondary heat treatment process by the method of manufacturing the glass-ceramic, disclosed in Korean Patent No. 10-1975548 by the applicants of the present disclosure, described above. However, the obtained glass-ceramics also have the same physical properties as one block itself. Thus, to realize deep colors like natural teeth using the glass-ceramic, a method of combining a plurality of resulting products is necessary to be applied. In other words, instant implementation of a natural-colored tooth by directly applying the machining method, such as CAD/CAM, using the bulk block itself is not easy.

The applicants of the present disclosure improved such problems and filed an application for a bulk block that is useful in manufacturing artificial tooth prostheses similar to natural teeth, which not only can reduce the time and process required for manufacturing an artificial tooth prosthesis but also can obtain the effect of enhancing structural stability in terms of force distribution based on functionally graded mechanical properties. The bulk block is currently registered (under Korean Patent No. 10-2246195) .

The present disclosure has been designed to provide a gradational bulk block having improved aesthetic properties, such as transmittance and shade, being further similar to natural teeth in terms of physical properties and the like, and having improved machinability.

### Disclosure

### Technical Problem

The present disclosure aims to provide a dental bulk block for machining, which can be used in manufacturing an artificial tooth restorative material exhibiting a multi-gradation of transmittance and physical properties similar to those of natural teeth so that repeatability and reproducibility are obtained by machining, such as CAD/CAM, without the addition of other processes.

The present disclosure also aims to provide a dental bulk block for machining, which not only can reduce the time and process for manufacturing an artificial tooth prosthesis but also can obtain the effect of enhancing structural stability in terms of force distribution based on functionally graded mechanical properties.

The present disclosure also aims to provide a method of simply manufacturing a dental bulk block for machining, which can be used in manufacturing an artificial tooth restorative material exhibiting a multi-gradation of transmittance and physical properties similar to those of natural teeth.

The present disclosure also aims to provide a method of easily manufacturing a dental restoration from a dental bulk block using a machining device.

### Technical Solution

One embodiment of the present disclosure provides a dental bulk block for machining, which is a glass-ceramic block including a crystalline phase in an amorphous glass matrix, the bulk block being a functionally graded material including crystalline phases in which among the crystalline phases, a main crystalline phase is lithium disilicate, and an additional crystalline phase includes quartz, lithium phosphate, and at least one selected from among cristobalite and tridymite, wherein the main crystalline phase has a size gradient with respect to a depth, and no interface is present at a point where a size gradient value of the main crystalline phase changes.

In one preferred embodiment of the present disclosure, the main crystalline phase may have a size gradient such that a mean particle diameter thereof is in a range of 0.05 um to 1.5 $\mu$m.

In the dental bulk block according to one embodiment of the present disclosure, an optical transmittance may have a gradient with respect to the depth.

In one preferred embodiment, the gradient of the optical transmittance may be in a range of 28% to 37% at a wavelength of 550 nm.

In one preferred embodiment, the gradient of the optical transmittance may be exhibited within a depth range of 0.5 mm.

In one preferred embodiment, the gradient of the optical transmittance may be exhibited within a depth range of 0.31 mm.

In the dental bulk block according to one embodiment of the present disclosure, L*, a*, and b* values obtained according to colorimetric analysis may have gradients with respect to the depth, and a color deviation ($\Delta$E) value may be exhibited within a depth range of 0.31 mm.

In the dental bulk block according to one preferred embodiment, a crystallinity may be in a range of 35% to 70%.

In the dental bulk block according to one embodiment of the present disclosure, a biaxial flexural strength may have a gradient with respect to the depth.

In one preferred embodiment, the gradient of the biaxial flexural strength may be in a range of 280 to 450 MPa.

The dental bulk block, according to one embodiment of the present disclosure, may include a continuous glass matrix.

In one preferred embodiment, the glass matrix may contain 69.0 to 75.0 wt% of $SiO_2$, 12.0 to 14.0 wt% of $Li_2O$, 2.5 to 5.5 wt% of $Al_2O_3$, 0.23 to 0.6 wt% of $ZnO$, 2.8 to 3.5 wt% of $K_2O$, 0.3 to 1.0 wt% of $Na_2O$, and 2.0 to 6.0 wt% of $P_2O_5$, in which a molar ratio of $Al_2O_3$ to $K_2O$ plus $ZnO$ may satisfy a value of 1.0 to 1.6.

Another embodiment of the present disclosure provides a method of manufacturing a dental bulk block for machining, the method including: manufacturing a block in a predetermined form by melting a glass composition containing 69.0 to 75.0 wt% of $SiO_2$, 12.0 to 14.0 wt% of $Li_2O$, 2.5 to 5.5 wt% of $Al_2O_3$, 0.23 to 0.6 wt% of $ZnO$, 2.8 to 3.5 wt% of $K_2O$, 0.3 to 1.0 wt% of $Na_2O$, and 2.0 to 6.0 wt% of $P_2O_5$, in which a molar ratio of $Al_2O_3$ to $K_2O$ plus $ZnO$ satisfies a value of 1.0 to 1.6, shaping and cooling the composition in a mold, and annealing the composition from a temperature of 480°C to 280°C for 20 minutes to 2 hours at a set rate; and

performing heat treatment on the block in a temperature range of 760°C to 880°C while giving a temperature gradient in a depth direction of the block.

In the method of manufacturing the dental bulk block according to one preferred embodiment, the heat treatment may be performed in such a manner that an upper portion of the block is heated to a temperature range of 840°C to 880°C, and a lower portion of the block is heated to a temperature range of 760°C to 800°C.

In one preferred embodiment, the heat treatment may be performed in a gradient heating furnace at an operating temperature of 900°C to 1,100°C for 1 to 40 minutes.

One embodiment of the present disclosure also provides a method of manufacturing a dental restoration, the method including: preparing a predetermined dental restoration by machining the dental bulk block in one of the embodiments using a machining device; and polishing or glazing the dental restoration.

In the method of manufacturing the dental restoration according to one preferred embodiment, the glazing of the dental restoration may be performed at a temperature of 730°C to 820°C for 30 seconds to 10 minutes.

The glazing of the dental restoration, which falls within the scope described above, may correspond to typical glazing that falls within a scope that does not impair the unique transmittance of the dental restoration obtained from the previous step.

In another example, the glazing of the dental restoration may be performed as a step of partially controlling the optical transmittance of the dental restoration obtained from the previous step according to the user's need. In this respect, the glazing of the dental restoration may be performed to control the transmittance of the machined dental restoration, using heat treatment at a temperature of at least 825°C. In this case, the glazing of the dental restoration is performed preferably at a temperature of at least 825°C for 1 to 20 minutes. In particular, the optical transmittance is reduced at a temperature over 840°C. Thus, to further reduce the optical transmittance, the glazing temperature may be further increased so that additional crystallization is partially allowed to occur at the same time.

The bulk block of the present disclosure is easily applicable to the manufacturing of an artificial tooth restorative material with a multi-gradation of transmittance and physical properties similar to those of natural teeth. As the dental restoration having controlled transmittance is obtainable by a simple heat treatment method on the user's side, there is an advantage of contributing to the simplification of logistics management.

**Advantageous Effects**

A dental bulk block, according to the present disclosure, can be used in manufacturing an artificial tooth restorative material with a multi-gradation of transmittance and physical properties similar to those of natural teeth so that repeatability and reproducibility are obtained by machining, such as CAD/CAM, without the addition of other processes. In addition, the dental bulk block not only can reduce the time and processes required for manufacturing an artificial tooth prosthesis but also can obtain the effect of enhancing structural stability in terms of force distribution based on functionally graded mechanical properties. Furthermore, such a dental bulk block has the advantage of being manufactured through a simple method of gradient heat treatment using a single glass composition with specific composition.

**Description of Drawings**

FIG. 1 is a graph showing X-ray diffraction results of a bulk block of the present disclosure;
FIG. 2 shows scanning electron microscope (SEM) images of the microstructure and crystalline phase size for each depth of a bulk block of the present disclosure;
FIG. 3 is a graph showing visible light transmittance measurement results for 0.31 mm-thick slice specimens of a bulk block of the present disclosure;
FIG. 4 is a comparative graph showing cutting resistance results for a bulk block of the present disclosure;
FIG. 5 is a schematic diagram illustrating a method of manufacturing a dental bulk block of the present disclosure as one example;
FIG. 6 is a graph showing the particle size of a main crystalline phase for each depth of a bulk block obtained according to one embodiment of the present disclosure; and
FIG. 7 is a graph showing changes in biaxial flexural strength for each depth of a bulk block obtained according to one embodiment of the present disclosure.

**Mode for Invention**

The foregoing and further aspects of the present disclosure will become more apparent through the preferred embodiments described with reference to the accompanying drawing. Hereinafter, the embodiment of the present disclosure will be described in detail so that those skilled in the art can easily understand and reproduce the present disclosure.

A dental bulk block for machining of the present disclosure is a glass-ceramic block including a crystalline phase in an amorphous glass matrix, the bulk block being a functionally graded material including crystalline phases in which among the crystalline phases, a main crystalline phase is lithium disilicate, and an additional crystalline phase includes quartz, lithium phosphate, and at least one selected from among cristobalite and tridymite wherein the main crystalline phase has a size gradient with respect to a depth, and no interface is present at a point where a size gradient value of the main crystalline phase changes.

In the description above and below, the term main crystalline phase may be defined as a crystalline phase that accounts for at least 80 wt% of the entire crystalline phase, and the term additional crystalline phase may be defined as the remaining crystalline phase other than the main crystalline phase among the entire crystalline phase.

The content of the crystalline phase is calculable through X-ray diffraction analysis. For example, in a specimen made up of two polymorphs, a and b, $F_a$, the ratio of crystalline phase a, is quantitatively expressed by Equation 1.

[Equation 1]

$$F_a = \frac{1}{1 + K\left(\dfrac{I_b}{I_a}\right)}$$

This value is obtainable by measuring the intensity ratio of the two crystalline phases and obtaining constant K. K is the absolute intensity ratio of the two pure polymorphs, $I_{oa}/I_{ob}$, and is obtained by measuring a standard material.

In the description above and below, the term main crystalline phase may be defined as being set based on the content calculated according to this method.

In addition, the expression "the main crystalline phase has a size gradient with respect to the depth" means that when plotting the size of the main crystalline phase by the depth of the bulk block on a graph, there is a slope of changes in the size of the main crystalline phase. In other words, this means that the size of the main crystalline phase is expressed in the form of a gradation with respect to the depth of the bulk block.

In addition, the expression "point where the size gradient value of the main crystalline phase changes" refers to the point where a substantial fluctuation occurs in the slope value of changes in the size of the main crystalline phase when plotting the size of the main crystalline phase by the depth of the bulk block on a graph. The term "substantial fluctuation" herein may mean a change represented by a single value but also includes a substantial change in light of the distribution of the value.

In addition, the expression "no interface is present at the point where the size gradient value of the main crystalline phase changes" may be interpreted as there is no significant boundary indicating interlayer separation at the depth point of the bulk block where changes in the slope value of the size of the main crystalline phase are exhibited. In other words, this means that in the bulk block, the main crystalline phase has a size gradient in a continuous form free of an interface by the depth.

On the other hand, the term "functionally graded material (FGM)" typically refers to a material in which the properties of the constituent material continuously change from one side to the other. In the present disclosure, even though there is practically no interface, the expression "functionally graded material" is adopted from the aspect that the properties of the constituent material continuously change.

In the description above and below, the bulk block is not limited in the shapes thereof and may, for example, include bulk materials having various shapes, such as block-like shapes, disk-like shapes, ingot-like shapes, and cylinder-like shapes.

In the bulk block according to the present disclosure, the main crystalline phase is lithium disilicate, and the additional crystalline phase includes quartz, lithium phosphate, and at least one selected from among cristobalite and tridymite. Any crystalline phases other than those described above are not included.

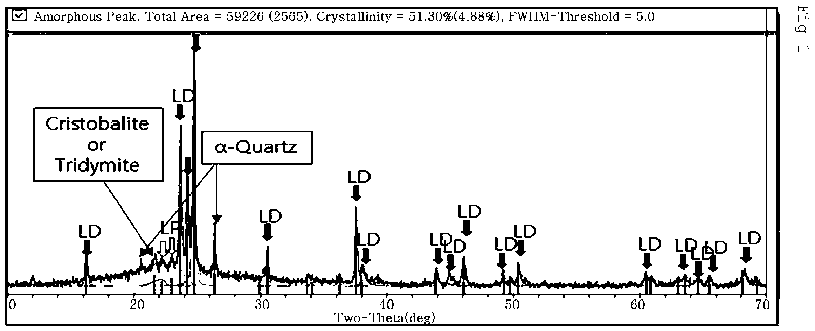

FIG. 1 shows a graph that is the same as the one showing XRD analysis results for the bulk block, according to one preferred embodiment.

In FIG. 1, the main crystalline phase of the dental bulk block, according to one embodiment of the present disclosure, is lithium disilicate. In addition, as one additional crystalline phase, a main peak appears at a $2\theta$ angle of 21.7 (degree), which may be interpreted as a polymorph of $SiO_2$, cristobalite (JCPDS #39-1425, the main peak observed at a 20 angle of 21.83 (degree) or tridymite (JCPDS #01-0378, at a $2\theta$ angle of 21.76 (degree)). As another additional crystalline phase, main peaks appear at 20 angles of 20.8 and 26.3 (degree), which may be interpreted as a polymorph of $SiO_2$, low-temperature quartz (a-quartz, JCPDS #83-0539, the main peak observed at $2\theta$ angles of 20.8 and 26.3 (degree)). As a further additional crystalline phase, main peaks appear at 20 angels of 22.1 and 22.9 (degree), which may be defined as lithium phosphate (JCPDS #15-0760, the main peaks observed at $2\theta$ angles of 22.3 and 23.1 (degree)) .

In the above and below descriptions, the XRD analysis may be interpreted as the analysis results obtained using an X-ray diffractometer (D/MAX-2500, Igaku, Japan; Cu K$\alpha$ (40 kV, 60 mA), injection speed: 6°/min, $2\theta$: 10 to 70 (degree), Rigaku, Japan).

These crystalline phases may be formed into microcrystals, which exhibit a wide range of sizes and size distributions with varying temperatures and are characterized by enabling wide ranges of mechanical properties and optical transmittance.

In addition, as the main crystalline phase has a size gradient with respect to the depth, the bulk block may enable a gradation of transmittance and mechanical properties with respect to the depth. Furthermore, as no interface is present at the point where the size gradient value of the main crystalline phase changes, there is no need for machining through interlayer bonding, and the problem of layer separation during the machining may be resolved. In addition, an artificial tooth prosthesis having improved structural stability may be provided in terms of force distribution based on the functional gradation.

In the bulk block of the present disclosure, the main crystalline phase has a size gradient such that a mean particle diameter is in a range of 0.05 um to 1.5 $\mu$m.

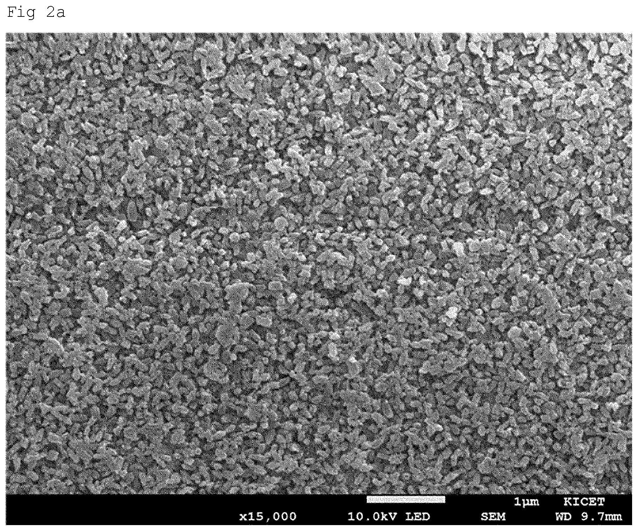

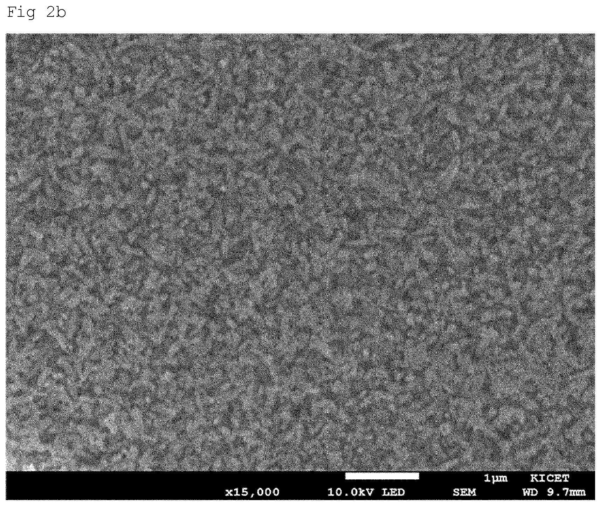

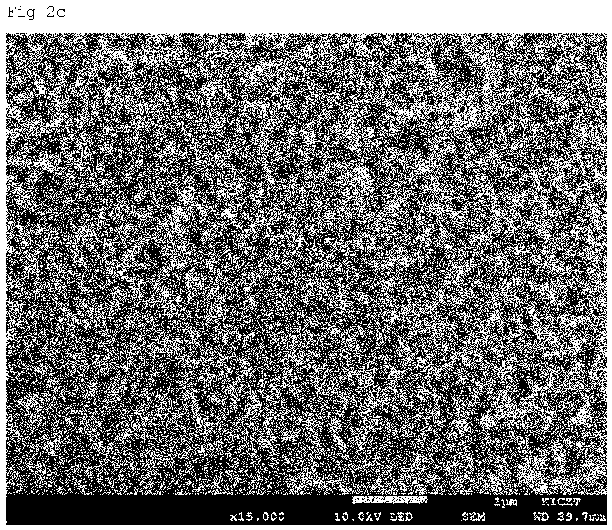

As one example, FIG. 2 shows scanning electron microscope (SEM) images of the dental bulk block of the present disclosure. FIG. 2A shows a low-temperature portion (bottom of the block) of the block subjected to gradient heat treatment, FIG. 2B shows an intermediate-temperature portion, and FIG. 2C shows a high-temperature portion (upper portion of the block). Specifically, FIG. 2A shows an SEM image of a lower portion of the block (at a depth of 20 mm), FIG. 2B shows an SEM image of an intermediate portion (at a depth of 10 mm), and FIG. 2C shows an SEM image of an upper portion of the block (at a depth of 0.5 mm).

The mean particle size of the crystalline phase may be derived through the SEM images obtained as described above, and may specifically be obtained according to the linear intercept method by drawing a diagonal or random straight line on the SEM images and dividing the number of crystalline phases through which the straight line passes by the length of the straight line with consideration of the magnification.

In the description above and below, the size of the crystalline phase is construed as being calculated according to this method.

The bulk block of the present disclosure is the functionally graded material. As the functionally graded material is applied to machining, for example, CAD/CAM, under the same machining conditions, the main crystalline phase preferably has a size gradient such that the mean particle diameter is in a range of 0.05 um to 1.5 um in the aspect that enables machinability to be taken into account and transmittance clinically available for artificial tooth restorative materials and the like to be exhibited.

In the dental bulk block of the present disclosure, as the main crystalline phase has a size gradient as described above, an optical transmittance has a gradient with respect to the depth.

In particular, considering the range of the mean particle diameter in terms of the crystalline size gradient described above, the gradient of the optical transmittance may be in a range of 28% to 37% at a wavelength of 550 nm.

In the description above and below, the optical transmittance is measured using a UV-visible spectrometer (UV-2401PC, Shimadzu, Japan).

As described above, in the dental bulk block of the present disclosure, no interface is present at the point where the size gradient value of the main crystalline phase changes. Thus, in this respect, it is confirmed that the gradient of the optical transmittance is exhibited within a depth range of 0.5 mm and substantially exhibited within a depth range of 0.31 mm.

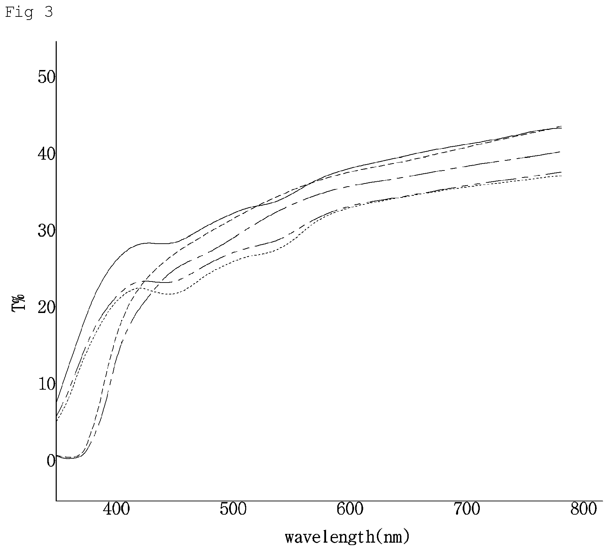

To measure the optical transmittance at each depth, the dental bulk block, according to the present disclosure, was sliced in the depth direction where the transparency decreases to prepare a specimen having a thickness of about 0.31 mm. Then, the surface of the specimen was cleaned using ethanol, and the optical transmittance was measured using a UV-visible spectrometer (UV-2401PC, Shimadzu, Japan). In this case, the wavelength for the measurement was set to be in a range of 300 to 800 nm, and the slit width was set to 2.0 nm. The results in FIG. 3 confirms that there is a difference in transmittance between the sliced specimens having a thickness of 0.31 mm.

In FIG. 3, each specimen corresponds to a specimen by depth in Table 1 below.

[Table 1]

| Specimen No. | Depth (mm) |
|---|---|
| 1 | 0.31 |
| 2 | 0.62 |
| 3 | 0.93 |
| 4 | 1.24 |
| 5 | 1.55 |

Such results may indicate that the optical transmittance value changes within a depth range of 0.31 mm, meaning that the gradient of transmittance is exhibited even at such thickness. This clearly shows that the dental bulk block of the present disclosure is the functionally graded material. In addition, from the results of the transmittance, it is seen that an artificial tooth having excellent aesthetics and appropriate concealing properties may be provided.

In another aspect, the dental bulk block of the present disclosure has a gradient in shade, and specifically, L*, a*, and b* values obtained according to colorimetric analysis have gradients with respect to the depth. As described above, in the dental bulk block of the present disclosure, no interface is present at the point where the size gradient value of the main crystalline phase changes. Thus, in this respect, it is confirmed that a color deviation (ΔE) value changes within a depth range of 0.31 mm.

Color standardization was necessary for accurate measurement, transmission, and reproduction of colors, so a color system was devised. Many color systems have been proposed, and the most widely used color system to date is the CIE L* a* b* color space (CIELAB color space) established by the Commission International de l'Eclairage (CIE) in 1976. In this case, L* represents lightness, and a* and b* represent chromaticity coordinates. In coordinates, the higher the L* value, the lighter the color, and the lower the L* value, the darker the color. In addition, a positive value of a* indicates red, a negative value of a* indicates green, a positive value of b* indicates yellow, and a negative value of b* indicates blue.

To measure the color of the dental bulk block, according to the present disclosure, at each gradient position, the dental bulk block was sliced in the depth direction where the transparency decreased to prepare a specimen having a thickness of about 0.31 mm. Then, the surface of the specimen was cleaned using ethanol, and the transmittance was analyzed using a UV-visible spectrometer (UV-2401PC, Shimadzu, Japan). In this case, the wavelength for the measurement was set to be in a range of 380 to 780 nm, and the slit width was set to 2.0 nm. After setting a baseline using a reference sample, the reflectance of each specimen was measured to obtain the L*a*b* color system. The L*, a*, and b* values were measured repeatedly three times to reduce errors, and then the mean value was used. Using these three values, ΔE, showing the color difference, was calculated. A ΔE value between the two specimens being 0 means that there is no color difference, and a value corresponding to 0 to 2 means that there is a slight color difference. A value of 2 to 4 means that the color difference is noticeably distinguishable, and a value of 4 to 6 means that the color difference is appreciably distinguishable. A value of 6 to 12 means that the color difference is much more noticeable, and a value of 12 or higher means that the color difference is much more noticeable.

As shown in FIGS. 1 and 2, regarding the dental bulk block, which is the glass-ceramic block including the crystalline phase in the amorphous glass matrix, the bulk block being the functionally graded material including the crystalline phases in which among the crystalline phases, the main crystalline phase is lithium disilicate, and the additional crystalline phase includes quartz, lithium phosphate, and at least one selected from among cristobalite and tridymite, wherein the main crystalline phase has a size gradient with respect to the depth, and no interface is present at the point where the size gradient value of the main crystalline phase changes, the results in Table 2 below confirms that the color deviation (ΔE) for each sliced specimen having a thickness of 0.31 mm appears to be in a range of 0.9 to 4.8 with respect the depth. Such results may indicate that the color deviation (ΔE) value changes within a depth range of 0.31 mm, meaning that the gradient of shade with varying colors is exhibited even at such thickness. This clearly shows that in another aspect, the dental bulk block of the present disclosure is the functionally graded material.

[Table 2]

| Specimen No. | Depth (mm) | L* | a* | b* | ΔE |
|---|---|---|---|---|---|
| 1 | 0.31 | 68.82 | -1.65 | 9.42 | |
| 2 | 0.62 | 70.80 | -1.71 | 11.50 | 4.7791 |
| 3 | 0.93 | 71.20 | -2.00 | 11.80 | 0.9949 |
| 4 | 1.24 | 72.10 | -2.40 | 12.00 | 1.2247 |
| 5 | 1.55 | 74.40 | -3.00 | 11.20 | 1.9235 |

In addition, in the dental bulk block of the present disclosure, a biaxial flexural strength has a gradient with respect to the depth. In particular, considering the range of mean particle diameter in terms of the crystalline size gradient, the biaxial flexural strength gradient may fall within a range of 280 MPa to 450 MPa. On the other hand, in terms of enabling functional gradients of a wide range of physical properties and in consideration of machinability, as described above, the dental bulk block of the present disclosure preferably has a crystallinity of 35% to 70%.

In the description above and below, the term "crystallinity" may be defined as a ratio of the crystalline phase to the amorphous glass matrix, which is obtainable through various methods. In one embodiment of the present disclosure, the crystallinity is a value that is automatically calculated through an X-ray diffractometer.

The dental bulk block of the present disclosure is the glass-ceramic in which the crystalline phase is precipitated from a continuous amorphous glass matrix, enabling the functionally graded material including the crystalline phases, in which among the crystalline phases, the main crystalline phase is lithium disilicate, and the additional crystalline phase includes quartz, lithium phosphate, and at least one selected from among cristobalite and tridymite, the main crystalline phase has a size gradient with respect to the depth, and no interface is present at a point where the size gradient value of the main crystalline phase changes, to be obtained.

In the description above and below, the term "continuous glass matrix" may be defined as one in which no interlayer interface is present in the glass matrix, and the composition constituting the glass matrix is the same throughout the entire block.

A preferred glass matrix specifically contains 69.0 to 75.0 wt% of $SiO_2$, 12.0 to 14.0 wt% of $Li_2O$, 2.5 to 5.5 wt% of $Al_2O_3$, 0.23 to 0.6 wt% of $ZnO$, 2.8 to 3.5 wt% of $K_2O$, 0.3 to 1.0 wt% of $Na_2O$, and 2.0 to 6.0 wt% of $P_2O_5$, in which a molar ratio of $Al_2O_3$ to $K_2O$ plus $ZnO$ satisfies a value of 1.0 to 1.6.

The glass composition precipitates the crystalline phase from the amorphous glass matrix through crystal nucleation and crystal growth heat treatment for crystallization, and the temperature of the glass matrix described above, at which the crystal grows, corresponds to 760°C to 880°C. In other words, crystal nuclei begin to be formed at a minimum temperature of 490°C, and the crystal grows as the temperature increases. Such crystal growth exhibits the lowest optical transmittance when used as an artificial tooth at a maximum temperature of 880°C. That is, the transmittance gradually decreases from the temperature at which the crystal grows to the maximum temperature of 880°C. Thus, considering such crystal growth, when being implemented in one bulk block, this may mimic the multi-gradation of natural teeth.

Not only a tooth itself but also all teeth of natural teeth have various transmittance, so when the changes in transmittance with varying heat treatment temperatures are allowed to be embodied in one bulk block, the multi-gradation of natural teeth may be sufficiently realizable.

From this point of view, the present disclosure provides a method of manufacturing a dental bulk block for machining, the method including: manufacturing a block in a predetermined form by melting a glass composition containing 69.0 to 75.0 wt% of $SiO_2$, 12.0 to 14.0 wt% of $Li_2O$, 2.5 to 5.5 wt% of $Al_2O_3$, 0.23 to 0.6 wt% of $ZnO$, 2.8 to 3.5 wt% of $K_2O$, 0.3 to 1.0 wt% of $Na_2O$, and 2.0 to 6.0 wt% of $P_2O_5$, in which a molar ratio of $Al_2O_3$ to $K_2O$ plus $ZnO$ satisfies a value of 1.0 to 1.6, shaping and cooling the composition in a mold, and annealing the composition from a temperature of 480°C to 280°C for 20 minutes to 2 hours at a set rate;

and performing heat treatment on the block in a temperature range of 760°C to 880°C while giving a temperature gradient in a depth direction of the block.

As described above, the glass composition may be characterized in that the optical transmittance of the material varies depending on the range of heat treatment temperature. Thus, when heat treatment is applied uniformly to the entire block, each block may exhibit uniform transmittance. However, when heat treatment is applied to the block while giving the temperature gradient, each block may exhibit a multi-gradation of physical properties or transmittance.

Bulk-form blocks are used as workpieces for machining, such as CAD/CAM. In the manufacturing method of the present disclosure, when performing the heat treatment on the block, heat is applied in the depth direction while giving the temperature gradient, enabling the bulk block with a multi-gradation of transmittance and physical properties to be manufactured.

Existing glass-ceramics typically have a large crystal size, making control of transmittance difficult, and the strength thereof is also strong, making the processing difficult. However, the glass composition adopted in the present disclosure enables the formation of microcrystals, which have various sizes and size distributions depending on temperature and each independently exhibit wide ranges of physical properties and optical transmittance. Thus, based on this, through the method of manufacturing the block from a single glass composition and then performing heat treatment while giving the temperature gradient, each bulk block is embodied so that a multi-gradation of mechanical properties and optical transmittance is achieved.

In this case, the expression "performing the heat treatment while giving the temperature gradient in the depth direction of the block" means that a sequentially increased temperature gradient may be given in the depth direction of the block from the bottom portion to the top portion, and a temperature gradient in a manner of partially giving a temperature difference is also acceptable. The selection of the temperature gradient method may vary depending on the characteristics of the natural teeth of the patient in need of an artificial tooth prosthesis or is variable depending on the unique characteristics of a tooth part in need of the prosthesis.

However, considering common natural teeth, the gradient of the heat treatment temperature is preferably given such that the heat treatment is performed while giving the temperature gradient in a manner that the temperature gradually increases from the lower portion to the upper portion with respect to the depth of the block.

In one preferred example, the heat treatment is performed in such a manner that an upper portion of the block is heated to a temperature range of 840°C to 880°C, and a lower portion of the block is heated to a temperature range of 760°C to 800°C. In addition, the actual heat treatment for such a temperature gradient is preferably performed in a gradient heating furnace at an operating temperature of 900°C to 1,100°C for 1 to 40 minutes.

When using the heat treatment method of the present disclosure described above using the glass composition described above, the structural characteristics of natural teeth, in which transmittance is low in the cervical region and becomes higher when being closer to the incisal edge, are mimicable. For this reason, there is no need for separate characterizing when manufacturing prostheses as in the existing method, which may be economically beneficial.

In addition, the physical properties of natural teeth are characterized in that the enamel, the surface layer, has high flexural strength, and the dentin inside thereof has low strength, thereby serving to absorb and distribute external forces. Hence, in the present disclosure, the functionally graded material having a gradient of mechanical properties, especially biaxial flexural strength, is enabled due to the difference in microstructure with varying heat treatment depths. In addition, the physical properties are characterized by being reproducible extremely similarly to those of natural teeth.

Manufacturing a dental restoration using the dental bulk block, obtained according to the present disclosure, may achieve significant improvement in terms of machinability. As one specific example, one embodiment of the present disclosure provides a method of manufacturing a dental restoration, which includes: manufacturing a predetermined dental restoration by machining the dental bulk block described above using a machining device; and polishing or glazing the dental restoration.

In the description above and below, the dental restoration includes crowns, inlays, onlays, veneers, abutments, and the like.

The glazing of the dental restoration herein may be performed at a temperature of 730°C to 820°C for 30 seconds to 10 minutes, which may be a typical finishing step of heat treatment where there is little change in transmittance due to the heat treatment. Glazing is typically performed within the scope that does not change the unique transmittance of the bulk block. Additionally, during glazing heat treatment, microcracks on the surface are reduced (surface healing), thereby increasing the strength by more than 50%.

However, in one specific embodiment, in the method of manufacturing the dental restoration using the bulk block according to the present disclosure, the glazing of the dental restoration may be performed to control the transmittance of the machined dental restoration, using heat treatment at a temperature of at least 825°C. In other words, after machining the bulk block to manufacture the dental restoration, the glazing of the dental restoration may be performed to control the lightness by reducing the transmittance in the last finishing step.

When manufacturing a dental restoration through mechanical work on the processor's or user's side using a bulk block, there may be cases where the transmittance unintentionally changes to a high level. In this case, a typical lithium disilicate-based bulk block needs to undergo a process of discarding the manufactured bulk block, re-machining a bulk block satisfying the target transmittance from the bulk block through predetermined heat treatment, and then machining the bulk block into a dental restoration again. However, the bulk block according to the present disclosure, which is a specific bulk block including a microcrystalline phase, may exhibit the characteristic that the transmittance is controlled with varying heat treatment temperatures. For this reason, there is no need for re-machining. Additionally, in the finishing step of finalizing the workpiece machined into the dental restoration, the transmittance may be controlled through the glazing process under predetermined conditions. As a result, the colored tooth generated in the middle of the machining process of the dental restoration may be easily covered through the glazing.

The glazing for such purpose is preferably performed at a temperature of at least 825°C for 1 to 20 minutes.

Characteristically, in the case of the dental bulk block obtained according to the present disclosure, when manufactured using a machining device, the resistance generated in the tool during the manufacturing may be significantly reduced. In one specific example, regarding the dental bulk block (the present disclosure) as shown in FIGS. 1 and 2, which is the glass-ceramic block including the crystalline phase in the amorphous glass matrix, the bulk block being the functionally graded material including the crystalline phases in which among the crystalline phases, the main crystalline phase is lithium disilicate, and the additional crystalline phase includes quartz, lithium phosphate, and at least one selected from among cristobalite and tridymite, wherein the main crystalline phase has a size gradient with respect to the depth, and no interface is present at the point where the size gradient value of the main crystalline phase changes, cutting time was measure by rotating the bulk block having a size of 12 × 14 × 18 mm using a low-speed saw (IOMET low-speed saw, Buehler, Germany) and a diamond electroplated wheel (2514485H17, Norton, USA) at 250 RPM. In addition, cutting times for the most conventional lithium disilicate block (Rosetta SM, manufactured by HASS Corp), zirconia-reinforced lithium disilicate bulk block (Celtra Duo, manufactured by DentsplySiron), and lithium aluminosilicate (LAS)-reinforced disilicate bulk block (Nice, manufactured by Straumann) were measured in the same manner.

Cutting resistance (%) was calculated from each cutting time value obtained as described above. Specifically, the cutting time obtained for the conventional lithium disilicate block was set to 100%, and the cutting time was converted to a relative percentage to calculate each cutting resistance value.

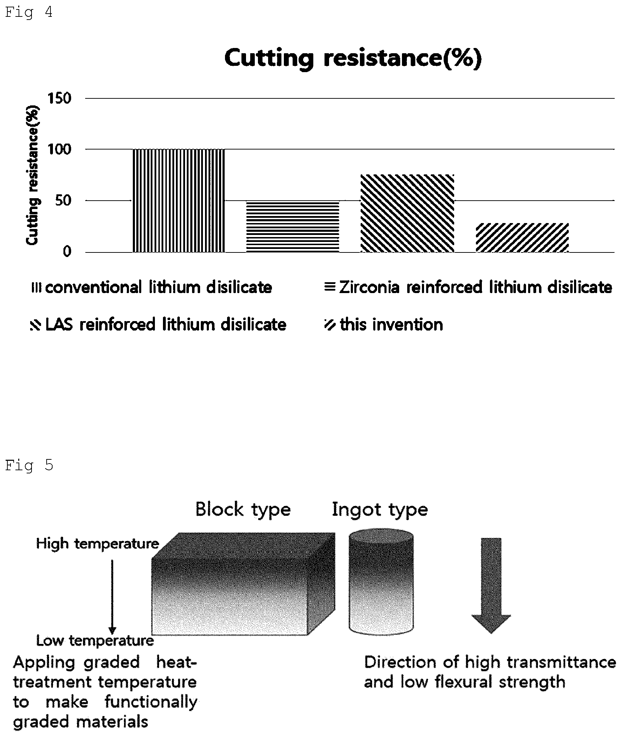

FIG. 4 shows the results thereof.

The results in FIG. 4 showed that the conventional lithium disilicate block had the highest cutting resistance, followed by the lithium aluminosilicate (LAS) glass-ceramic and the zirconia-reinforced glass-ceramic. Additionally, the block, according to the present disclosure, had the lowest cutting resistance. These results confirm that the glass-ceramic block of the present disclosure is the most machinable.

In one specific embodiment of the present disclosure, the glass composition containing 69.0 to 75.0 wt% of $SiO_2$, 12.0 to 14.0 wt% of $Li_2O$, 2.5 to 5.5 wt% of $Al_2O_3$, 0.23 to 0.6 wt% of ZnO, 2.8 to 3.5 wt% of $K_2O$, 0.3 to 1.0 wt% of $Na_2O$, and 2.0 to 6.0 wt% of $P_2O_5$, in which a molar ratio of $Al_2O_3$ to $K_2O$ plus ZnO satisfies a value of 1.0 to 1.6, is weighed and mixed.

When added to silicate glass, $Al_2O_3$ enters the tetrahedral site to act as a glass former and serve to increase the viscosity and reduce the ion mobility. On the other hand, $K_2O$ and ZnO reduce the viscosity and increase the ion mobility. As the ion mobility increases, the formation of cristobalite or tridymite may preferentially grow. In addition, an increase in modifiers, such as ZnO, increases the ion mobility. Here, when containing excessive amounts of $SiO_2$, minor crystalline phases, such as cristobalite or tridymite and quartz, are precipitated from the glass matrix along with lithium disilicate, the main crystalline phase. In this respect, the molar ratio of $Al_2O_3$ to $K_2O$ plus ZnO is preferably in a range of 1.0 to 1.6 in providing the bulk block of the present disclosure, which includes quartz and cristobalite or tridymite as the additional crystalline phase.

As for the glass composition, $Li_2CO_3$ may be added instead of $Li_2O$, and carbon dioxide ($CO_2$), which is the carbon (C) component of $Li_2CO_3$, is discharged in the form of a gas during a glass melting process. In addition, in the alkaline oxide, $K_2CO_3$ and $Na_2CO_3$ may be added instead of $K_2O$ and $Na_2O$, respectively, and carbon dioxide ($CO_2$), which is the carbon (C) component of $K_2CO_3$ and $Na_2CO_3$, is discharged in the form of a gas and escapes during the glass melting process.

Mixing is based on a dry mixing process, and as for the dry mixing process, a ball milling process and the like may be used. Looking into the ball milling process in detail, starting materials are charged into a ball milling machine, mechanically ground by rotating the ball milling machine at a constant speed, and uniformly mixed. The balls used in the ball milling machine may be made of ceramic materials, such as zirconia or alumina, and the balls may be the same in size or have at least two different sizes. Considering the target particle size, the ball size, milling time, rotation speed of the ball milling machine per minute, and the like are controlled. For example, considering the particle size, the ball size may be set to be in a range of about 1 mm to 30 mm, and the rotation speed of the ball milling machine may be set to be in a range of about 50 to 500 rpm. The ball milling is preferably performed for 1 to 48 hours in consideration of the target particle size and the like. Through the ball milling, the starting materials are ground to fine particles and allowed to have a uniform particle size while being mixed uniformly.

The mixed starting materials are placed in a melting furnace, and the melting furnace including the starting materials is then heated to melt the starting materials. The melting herein means that the starting materials are transformed into a material state with the viscosity of a liquid state, not a solid state. The melting furnace is preferably made of a material having a high melting point, high strength, and a low contact angle to prevent the molten material from sticking. To this end, the melting furnace is preferably made of platinum (Pt), diamond-like-carbon (DLC), and chamotte, or the surface of the melting furnace is preferably coated with a material, such as platinum (Pt) or diamond-like-carbon (DLC).

The melting is preferably performed at a temperature of 1,400°C to 2,000°C to the atmospheric pressure for 1 to 12 hours. When the melting point is lower than 1,400°C, the starting materials may be melted insufficiently, and when the melting point exceeds 2,000°C, excessive energy consumption is required, which is economically infeasible. Thus, the melting is performed preferably at a temperature that falls within the range described above. Additionally, when the melting takes an extremely short time, the starting material may be melted insufficiently, and when the melting takes an extremely long time, excessive energy consumption is required, which is economically infeasible. The heating rate of the melting furnace is preferably in a range of about 5°C/min to 50°C/min. However, when the heating rate of the melting furnace is extremely slow, the melting takes a long time, resulting in a decrease in productivity. When the heating rate of the melting furnace is extremely fast, a rapid increase in temperature results in an increase in the volatilization amount of the starting materials, which may be undesirable for the physical properties of the glass-ceramic. Thus, the temperature of the melting furnace is preferably increased at a heating rate that falls within the range described above. The melting is performed preferably in an oxidizing atmosphere, such as oxygen ($O_2$) or air.

The molten material is poured into a designated mold to obtain a dental glass-ceramic having the desired shape and size. The mold is preferably made of a material having a high melting point, high strength, and a low contact angle to prevent the glass molten material from sticking. To this end, the mold is preferably made of materials, such as graphite and carbon. In addition, to prevent thermal shock, the molten material is preferably poured into the mold after preheating the mold to a temperature of 200°C to 300°C.

The molten material included in the mold is shaped and cooled, so annealing is preferably performed from a temperature of 480°C to 280°C for 20 minutes to 2 hours at a set rate after the cooling process. Such annealing reduces the stress deviation in a molded article, preferably to the point where no stress exists, which may have a desirable effect on controlling the size of the crystalline phase and improving the homogeneity of crystal distribution in the subsequent crystallization. Accordingly, the ultimately desired functionally graded material may be obtainable.

The set rate herein is preferably in a range of 1.6°C/min to 10°C/min.

The molded article having undergone the annealing process as described above is transferred to a crystallization heat treatment sintering furnace for nucleation and crystal growth, thereby manufacturing the target glass-ceramic.

FIG. 5 schematically illustrates a method of performing crystallization heat treatment while giving the temperature gradient according to the present disclosure. When performing crystallization heat treatment on a block-type or ingot-type bulk block, the heat treatment is performed while giving the temperature gradient such that the upper portion is heated to high temperatures and the lower portion is heated to low temperatures in the depth direction.

In the above and below descriptions, the performing of the heat treatment while giving the temperature gradient is not particularly limited in a device or method. However, in one example, the heat treatment may be performed in a gradient heating furnace and is preferably performed at an operating temperature of 900°C to 1,100°C considering the heat treatment temperature.

Through the heat treatment with such a temperature gradient, in terms of the optical transmittance, a gradient of high transmittance is exhibited from the high-temperature portion to the low-temperature portion. Additionally, in terms of the biaxial flexural strength, a gradient of low flexural strength appears. This is because the crystal size in the glass-ceramic may be controlled with temperatures. The crystalline phase produced after the temperature-gradient heat treatment includes lithium disilicate and the additional crystalline phase, including quartz, lithium phosphate crystalline phase, and at least one selected from cristobalite and tridymite. In addition, the crystalline phase produced is allowed to have a size gradient such that a mean particle diameter is in a range of 0.05 um to 1.5 $\mu$m at a temperature gradient of 760°C to 880°C.

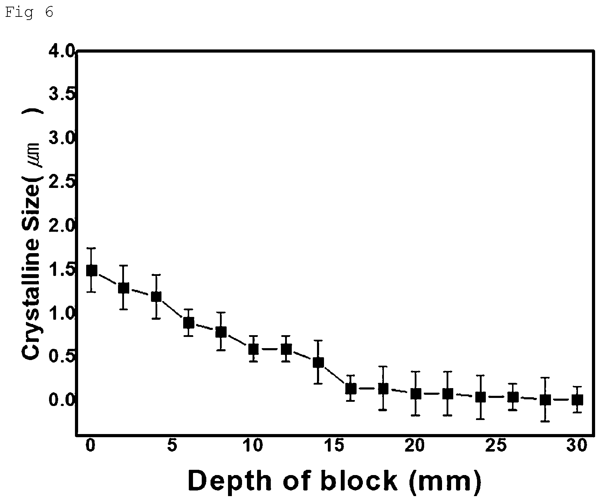

On the other hand, FIG. 6 shows the analysis results of the crystalline particle size with respect to the depth of the bulk block obtained according to the present disclosure.

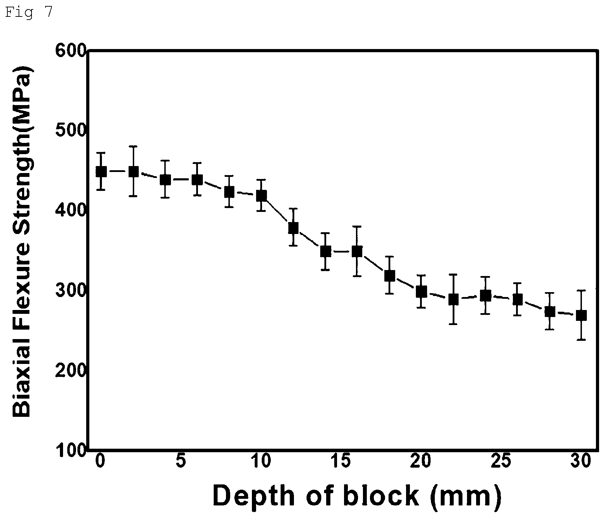

In addition, FIG. 7 shows the measurement results of changes in the biaxial flexural strength with respect to the depth of the bulk block obtained according to the present disclosure.

Although the present disclosure has been described with reference to one embodiment shown in the drawing, this is merely exemplary, and those skilled in the art will understand that various modifications and equivalent other embodiments are possible therefrom.

**Industrial Applicability**

The present disclosure relates to a dental bulk block for machining, which is useful in manufacturing artificial teeth having structural properties similar to those of natural teeth, and a method of manufacturing the same.

The dental bulk block, according to the present disclosure, can be easily used in manufacturing an artificial tooth restorative material with a multi-gradation of transmittance and physical properties similar to those of natural teeth so that repeatability and reproducibility are obtained by machining, such as CAD/CAM and the like, without the addition of other processes. In addition, the dental bulk block not only can reduce the time and processes required for manufacturing an artificial tooth prosthesis but also can obtain the effect of enhancing structural stability in terms of force distribution based on functionally graded mechanical properties. Furthermore, such a dental bulk block has the advantage of being manufactured through a simple method of gradient heat treatment using a single glass composition with specific composition.

**Claims**

1. A dental bulk block for machining, which is a glass-ceramic block comprising a crystalline phase in an amorphous glass matrix,

   the bulk block being a functionally graded material comprising crystalline phases, wherein among the crystalline phases, a main crystalline phase is lithium disilicate, and an additive crystalline phase comprises quartz, lithium phosphate, and at least one selected from among cristobalite and tridymite,
   wherein the main crystalline phase has a size gradient with respect to a depth, and
   no interface is present at a point where a size gradient value of the main crystalline phase changes.

2. The bulk block of claim 1, wherein the main crystalline phase has a size gradient such that a mean particle diameter thereof is in a range of 0.05 um to 1.5 um.

3. The bulk block of claim 1, wherein an optical transmittance has a gradient with respect to the depth.

4. The bulk block of claim 3, wherein the gradient of the optical transmittance is in a range of 28% to 37% at a wavelength of 550 nm.

5. The bulk block of claim 3, wherein the gradient of the optical transmittance is exhibited within a depth range of 0.5 mm.

6. The bulk block of claim 5, wherein the gradient of the optical transmittance is exhibited within a depth range of 0.31 mm.

7. The bulk block of claim 1, wherein L*, a*, and b* values obtained according to colorimetric analysis have gradients with respect to the depth, and
   a color deviation ($\Delta$E) value changes within a depth range of 0.31 mm.

8. The bulk block of claim 1, wherein a crystallinity is in a range of 35% to 70%.

9. The bulk block of claim 1, wherein a biaxial flexural strength has a gradient with respect to the depth.

10. The bulk block of claim 9, wherein the gradient of the biaxial flexural strength is in a range of 280 to 450 MPa.

11. The bulk block of claim 1, wherein the bulk block comprises a continuous glass matrix.

12. The bulk block of claim 1 or 11, wherein the glass matrix comprises 69.0 to 75.0 wt% of $SiO_2$, 12.0 to 14.0 wt% of $Li_2O$, 2.5 to 5.5 wt% of $Al_2O_3$, 0.23 to 0.6 wt% of ZnO, 2.8 to 3.5 wt% of $K_2O$, 0.3 to 1.0 wt% of $Na_2O$, and 2.0 to 6.0 wt% of $P_2O_5$, wherein a molar ratio of $Al_2O_3$ to $K_2O$ plus ZnO satisfies a value of 1.0 to 1.6.

13. A method of manufacturing a dental bulk block for machining, the method comprising:

    manufacturing a block in a predetermined form by

        melting a glass composition comprising 69.0 to 75.0 wt% of $SiO_2$, 12.0 to 14.0 wt% of $Li_2O$, 2.5 to 5.5 wt% of $Al_2O_3$, 0.23 to 0.6 wt% of ZnO, 2.8 to 3.5 wt% of $K_2O$, 0.3 to 1.0 wt% of $Na_2O$, and 2.0 to 6.0 wt% of $P_2O_5$, wherein a molar ratio of $Al_2O_3$ to $K_2O$ plus ZnO satisfies a value of 1.0 to 1.6,
        shaping and cooling the composition in a mold, and
        annealing the composition from a temperature of 480°C to 280°C for 20 minutes to 2 hours at a set rate; and

    performing heat treatment on the block in a temperature range of 760°C to 880°C while giving a temperature gradient in a depth direction of the block.

14. The method of claim 13, wherein the heat treatment is performed in such a manner that an upper portion of the block is heated to a temperature range of 840°C to 880°C, and a lower portion of the block is heated to a temperature range of 760°C to 800°C.

15. The method of claim 13 or 14, wherein the heat treatment is performed in a gradient heating furnace at an operating temperature of 900°C to 1,100°C for 1 to 40 minutes.

16. A method of manufacturing a dental restoration, the method comprising:

    manufacturing a predetermined dental restoration by machining the bulk block of claim 1 using a machining device; and
    polishing or glazing the dental restoration.

17. The method of claim 16, wherein the glazing of the dental restoration is performed at a temperature of 730°C to 820°C for 30 seconds to 10 minutes.

18. The method of claim 16, wherein the glazing of the dental restoration is performed to control the transmittance of the machined dental restoration, using heat treatment at a temperature of at least 825°C.

19. The method of claim 18, wherein the glazing of the dental restoration is performed at a temperature of at least 825°C for 1 to 20 minutes.

Fig 1

Fig 2a

Fig 2b

Fig 2c

Fig 3

Fig 4

# Cutting resistance(%)

III conventional lithium disilicate    ≡ Zirconia reinforced lithium disilicate

◊ LAS reinforced lithium disilicate    ⌀ this invention

Fig 5

Block type    Ingot type

High temperature

Low temperature

Appling graded heat-treatment temperature to make functionally graded materials

Direction of high transmittance and low flexural strength

Fig 6

Fig 7

**EP 4 349 301 A1**

## INTERNATIONAL SEARCH REPORT

| International application No. |
| --- |
| **PCT/KR2021/006675** |

### A. CLASSIFICATION OF SUBJECT MATTER

**A61C 13/00**(2006.01)i; **A61C 13/083**(2006.01)i; **A61K 6/802**(2020.01)i; **A61K 6/853**(2020.01)i; **A61L 27/10**(2006.01)i; **C04B 35/622**(2006.01)i; **C04B 35/14**(2006.01)i; **C04B 35/01**(2006.01)i

According to International Patent Classification (IPC) or to both national classification and IPC

### B. FIELDS SEARCHED

Minimum documentation searched (classification system followed by classification symbols)

A61C 13/00(2006.01); A61K 6/00(2006.01); A61K 6/02(2006.01); A61K 6/831(2020.01); C03C 10/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 치과용 벌크 블록(dental bulk block), 글라스 세라믹(glass ceramic), 리튬 디실리케이트(lithium disilicate), 크리스토발라이트(cristobalite), 트리디마이트(tridymite), 쿼츠(quartz), 리튬 포스페이트(lithium phosphate), 경사도(gradient), 광투과도(light transmittance), 온도구배(temperature gradient)

### C. DOCUMENTS CONSIDERED TO BE RELEVANT

| Category* | Citation of document, with indication, where appropriate, of the relevant passages | Relevant to claim No. |
| --- | --- | --- |
| Y | KR 10-2020-0137198 A (HASS CORPORATION) 09 December 2020 (2020-12-09)<br>See abstract; claims 1-13; paragraphs [0017], [0041] and [0063]-[0076]; and figures 1-7. | 1-19 |
| Y | KR 10-2018-0043335 A (IVOCLAR VIVADENT AG) 27 April 2018 (2018-04-27)<br>See abstract; claim 1; and paragraphs [0115]-[0116]. | 1-12,16-19 |
| Y | KR 10-2015-0137573 A (KOREA INSTITUTE OF CERAMIC ENGINEERING AND TECHNOLOGY et al.) 09 December 2015 (2015-12-09)<br>See abstract; and paragraph [0052]. | 13-15 |
| A | JUNG, S.-K. et al. Modulation of Lithium Disilicate Translucency through Heat Treatment. Materials. 21 April 2021, vol. 14, article no.: 2094, inner pp. 1-10.<br>See entire document. | 1-19 |
| A | KR 10-2019-0022678 A (CORNING INCORPORATED) 06 March 2019 (2019-03-06)<br>See entire document. | 1-19 |

☐ Further documents are listed in the continuation of Box C.　　☑ See patent family annex.

\* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **25 February 2022** | **25 February 2022** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

22

**INTERNATIONAL SEARCH REPORT**
Information on patent family members

International application No.

**PCT/KR2021/006675**

| Patent document cited in search report | | | Publication date (day/month/year) | Patent family member(s) | | | Publication date (day/month/year) |
|---|---|---|---|---|---|---|---|
| KR | 10-2020-0137198 | A | 09 December 2020 | AU | 2019-448021 | A1 | 23 December 2021 |
| | | | | CN | 110590163 | A | 20 December 2019 |
| | | | | EP | 3744696 | A1 | 02 December 2020 |
| | | | | JP | 2020-193191 | A | 03 December 2020 |
| | | | | JP | 6986285 | B2 | 22 December 2021 |
| | | | | US | 11104600 | B2 | 31 August 2021 |
| | | | | US | 2020-0377403 | A1 | 03 December 2020 |
| | | | | WO | 2020-241974 | A1 | 03 December 2020 |
| KR | 10-2018-0043335 | A | 27 April 2018 | CA | 2995309 | A1 | 02 March 2017 |
| | | | | CA | 2995309 | C | 16 June 2020 |
| | | | | CN | 108025949 | A | 11 May 2018 |
| | | | | DE | 202015009943 | U1 | 12 October 2021 |
| | | | | EP | 3135641 | A1 | 01 March 2017 |
| | | | | JP | 2018-526317 | A | 13 September 2018 |
| | | | | JP | 2021-066658 | A | 30 April 2021 |
| | | | | JP | 6907192 | B2 | 21 July 2021 |
| | | | | KR | 10-2020-0086382 | A | 16 July 2020 |
| | | | | KR | 10-2174282 | B1 | 05 November 2020 |
| | | | | US | 10501366 | B2 | 10 December 2019 |
| | | | | US | 2018-0244564 | A1 | 30 August 2018 |
| | | | | US | 2020-0079685 | A1 | 12 March 2020 |
| | | | | WO | 2017-032745 | A1 | 02 March 2017 |
| KR | 10-2015-0137573 | A | 09 December 2015 | KR | 10-1621886 | B1 | 18 May 2016 |
| KR | 10-2019-0022678 | A | 06 March 2019 | CN | 109476532 | A | 15 March 2019 |
| | | | | EP | 3475235 | A1 | 01 May 2019 |
| | | | | EP | 3475236 | A1 | 01 May 2019 |
| | | | | JP | 2019-522620 | A | 15 August 2019 |
| | | | | TW | 201815715 | A | 01 May 2018 |
| | | | | US | 2019-0177210 | A1 | 13 June 2019 |
| | | | | US | 2019-0256407 | A1 | 22 August 2019 |
| | | | | US | 2021-0179484 | A1 | 17 June 2021 |
| | | | | WO | 2017-223551 | A1 | 28 December 2017 |
| | | | | WO | 2017-223561 | A1 | 28 December 2017 |

Form PCT/ISA/210 (patent family annex) (July 2019)

## REFERENCES CITED IN THE DESCRIPTION

**Patent documents cited in the description**

- KR 101975548

- KR 102246195

**Non-patent literature cited in the description**

- **MARCUS P. BOROM ; ANNA M. TURKALO.** The Pacific Coast Regional Meeting. The American Ceramic Society, 31 October 1973